# EUROPEAN PATENT SPECIFICATION

(11) **EP 1 165 610 B1**
(45) Date of publication and mention of the grant of the patent: **11.01.2006**
(21) Application number: 99915734.0
(22) Date of filing: 29.03.1999
(51) Int. Cl.: C07K 14/47, A61K 38/17

(54) **PEPTIDE USEFUL IN TREATING MULTIPLE SCLEROSIS AND A PHARMACEUTICAL COMPOSITION COMPRISING THE SAME**
PEPTID ZUR BEHANDLUNG VON MULTIPLER SCLEROSE UND SEINE PHARMAZEUTISCHE ZUSAMMENSETZUNG
PEPTIDE UTILE AU TRAITEMENT DE LA SCLEROSE EN PLAQUE ET COMPOSITION PHARMACEUTIQUE CORRESPONDANTE

(43) Date of publication of application: 02.01.2002
(73) Proprietor: TECNOGEN S.C.P.A., 81015 Piana di Monte Verna (Caserta) (IT)
(72) Inventor: MARINO, Maria, I-81100 Caserta (IT); IPPOLITO, Agostino, I-80145 Napoli (IT); FASSINA, Giorgio, I-20133 Milano (IT)
(74) Representative: Marchi, Massimo
(86) International application number: PCT/EP1999/002268
(87) International publication number: WO 2000/058354

(56) References cited:
- EP-A- 0 779 297
- BROCKE S ET AL: "TREATMENT OF EXPERIMENTAL ENCEPHALOMYELITIS WITH A PEPTIDE ANALOGUE OF MYELIN BASIC PROTEIN" NATURE,GB,MACMILLAN JOURNALS LTD. LONDON, vol. 379, no. 6563, page 343-346 XP000601452 ISSN: 0028-0836 cited in the application
- KARIN E.A.: "Short peptide-based tolerogens without self-antigenic or pathogenic activity reverse autoimmune disease " JOURNAL OF IMMUNOLOGY, vol. 160, no. 10, 15 October 1998 (1998-10-15), pages 5188-5194, XP002123434 BALTIMORE US

### SEQUENCE LISTING

<110> Tecnogen S.C.p.A.
<120> A peptide compound useful in treating the multiple sclerosis and a pharmaceutical composition comprising the same
<130> Tecno 9
<140>
   <141>
<160> 5
<170> PatentIn Ver. 2.0
<210> 1
   <211> 20
   <212> PRT
   <213> Artificial Sequence
<220>
   <223> Description of Artificial Sequence: peptide
<400> 1
   Asn Gly Val Gly His Gly Phe Gly Asn Gly Val Gly Pro Gly Thr Gly
   1 5 10 15
   Pro Gly Ser Gly
   20
<210> 5
   <211> 20
   <212> PRT
   <213> Artificial Sequence
<220>
   <223> Description of Artificial Sequence: peptide corresponding to 81-100 sequence of the encephalitogenic mouse myelin basic protein epitope
<400> 5

## Description

This invention relates to a peptide compound useful in treating the Multiple Sclerosis and a pharmaceutical composition comprising the same.

Multiple Sclerosis (MS) is the most common acquired demyelinating disease of the central nervous system. In particular, MS is characterized pathologically by plaques or islands of demyelination disseminated through the Central Nervous System (CNS), primarily in the white matter, the optic nerves, and periventricular areas.

In fact the loss of myelin, which wraps up neurons of CNS, is accompanied by a disruption in the ability of the nerves to conduct electrical impulses to and from the brain, and this produces the various symptoms of MS. Such symptoms depend on which areas of the central nervous system have been affected. The systems commonly affected include the vision, co-ordination, sensation, cognitive functions, bladder control, speech with slow enunciation with a tendency to hesitate at the beginning of a word or syllable, but also paresthesias in one or more extremities, in the trunk, or on one side of the face, weakness or clumsiness of a leg or a hand.

The course is highly varied and unpredictable and in most patients, remittent. Some people are minimally affected by the disease while others have rapid progress to total disability.

In addition, it is known that women are more likely to develop MS than men and the disease is more common in temperate climates (1:2000) than in the tropics (1:10,000).

Presently a therapy capable of stopping the progression of primary neurologic disability caused by MS is not known. Current therapy favors corticosteroids, which accelerate recovery from acute attacks, but are not believed to alter the long-term course. Immunosuppressants, as cyclophosphamide, leflunomide and cyclosporine are sometimes used in progressive MS, but they are teratogenic and carcinogenic.

For this reasons scientists are now involved in the search of new therapeutic agents capable of defusing destructive immune cells, leaving the rest of the body's protective immune system intact.

So far, such studies indicate a more clear role carried on by T lymphocytes. T lymphocytes are able to distinguish between substances belonging to the body (self) and substances that are foreign. They recognize one specific antigen (normally a peptide fragment) through a protein protruding from the T cell surface, the T cell receptor (TCR), only when this fragment is associated with a molecule of the major histocompatibility complex (MHC), localized on the surface of specialized antigen presenting cells. The trimolecular complex formed (MHC-peptide-TCR) seems to be responsible for activation of an autoreactive T cell clone, which releases proteins as interferon y and the tumor necrosis factor, that are responsible for MS demyelinating process.

It is known that T cell receptor can recognize modified ligands and respond in a variety of ways through different mechanisms as TCR antagonism (De Magistris et al., "Cell", 68, 625-634, 1992 ), T cell tolerance or T cell anergy (Schwartz, "Science", 248, 1349-1356, 1990; Sloan-Lancaster et al., "Nature", 363, 156-159, 1993).

Apparently that anergy can be induced by synthetic peptides corresponding to the major immunodominant T cell determinants of native protein antigens, which are able to induce T cell unresponsiveness to themselves (Vidard et al., "Proc. Natl. Acad. Sci. USA" 92, 2259-2262, 1995). Anergic lymphocytes are incapable of producing IL-2 on restimulation with antigen (Schwartz, "Science", 248, 1349-1356, 1990).

For example, substitution of a proline (96→Pro) with an alanine in the encephalitogenic peptide epitope 87-99 Val-His-Phe-Phe-Lys-Ile-Val-Thr-Pro-Arg-Thr-Pro from human Myelin Basic Protein (hMBP), caused T cell hyporesponsiveness, with a reduced ability to induce a proliferative response in a T cell clone specific for MBP. However, such peptide is still capable of causing encephalomyelitis in a susceptible mouse strain (Broke, S., et al., "Nature", 379, 343-346, 1996).

This approach did not lead to clinically useful results since altered peptides which are able to specifically act on the autoreactive T cell subset, may cause in certain conditions also the disease.

For these reasons, it is still felt the need of a peptide capable of inducing a state of unresponsiveness (anergy) of autoreactive T lymphocytes in susceptible people or in individuals affected by MS without inducing an autoreactive response.

Now it has been found that these properties are owned by a peptide compound of formula (I):
where
R is H- or COCH₃, R' is COOH or CONH₂ and
each amino acid has L or D configuration.

It is therefore a first object of the present invention to provide a peptide compound according to claim 1.

The peptide compound of this invention may be prepared according to conventional methods used in peptide chemistry for suitable protection of amino acids, coupling of protected amino acids and removal of protective groups.

Preferably said peptide is prepared according to solid-phase synthesis techniques, which comprise the following steps:
- coupling, by means of a suitable reagent, of the first C-terminal amino acid protected both at the alpha amino group and amino group, if any, at the side chain to a suitable support for solid phase synthesis;
- removal of the alpha amino protective by means of suitable reagent;
- coupling of the second amino acid, starting from the C-terminal end, protected both at the alpha amino group and at the side chain amino group, if any;
- removal of the protective group at the alpha amino group and performance of the coupling and deprotection steps until completion of the couplings of all the amino acids encompassed in the peptide sequence up to the N-terminal residue;
- removal of the remaining side chain protective groups and detachment of the assembled peptide from the support for solid phase synthesis.

These techniques are widely described in the literature and are well known to the person skilled in the art (Atherton & Sheppard, 1989, Solid Phase Peptide Synthesis, IRL Press, Oxford, UK).

It is therefore a second object of the present invention to provide a pharmaceutical composition, according to claim 2.

Preferably, the pharmaceutical composition according to this invention is prepared in a suitable dosage form comprising an effective dose of a peptide compound of formula (I) (SEQ ID NO: 1) and at least a pharmaceutically acceptable inert ingredient.

Examples of suitable administration routes are the inhalation or oral route; as they are known to induce a state of immunologic hyporesponsiveness (oral tolerance). (Grainstein, et al., "Chem. Immunol.", 58, 259-290, 1994).

Typical examples of suitable dosage forms are pills, capsules, sugar coated pills, granules, solutions and syrups for oral administration, unguents and plasters for topic administration; suppositories for rectal administration and sterile solutions for injectable, inhalation and ophthalmic administration.

The dosage forms may also contain other conventional ingredients like preservatives, stabilizers, surface-active agents, buffers, salts to regulate the osmotic pressure, emulsifying agents, sweeteners, dyes, flavors and so on.

When required by particular therapies, the pharmaceutical composition of this invention may contain other active pharmacological ingredients whose concomitant administration is therapeutically useful.

Typically the amount of the peptide compound of this invention in the pharmaceutical composition of the invention will be such that it insures an administration level of from 1 to 100 mg/Kg/day.

The amount of a peptide compound of formula (I) (SEQ ID NO: 1) in a pharmaceutical composition of this invention may vary in a rather wide range depending on known factors such as, for example, the severity of the disease, the body weight of the patient, the dosage form, the chosen route of the administration, the number of dosage forms administrated daily and the specific efficacy of the chosen peptide compound of formula (I) (SEQ ID NO: 1).

The dosage forms of the pharmaceutical composition of this invention can be prepared according to techniques which are known to the pharmaceutical chemist and comprise procedures such as mixing, granulation, compression, dissolution, sterilization and so on.

As described in detailed below (Assay 1), the biological activity of the peptide compound of this invention has been evaluated by *in vivo* studies on a susceptible strain of mice suitable to develop experimental autoimmune encephalomyelitis (EAE), which represents a murine model of MS.

Indeed, both MS and EAE are a CD4⁺ T cell mediated disease and can be induced by simply immunization of mice with MBP or with one of its encephalitogenic peptide fragments. EAE, as MS, is characterized by invasion of the central nervous system by T lymphocytes, resulting in demyelination and acute, chronic or chronic relapsing paralysis.

Assay 1 shows that peptide compound of the present invention is able to delay the onset of clinical signs of the disease, diminishing the severity and increasing the survival rate of treated mice.

The present invention is further described by the following Examples and Assays.

### EXAMPLE 1

### Peptide Compounds of formula (I) (SEQ ID NO: 1) and Comparison Peptide Compound P81-100 (SEQ ID NO: 5) (corresponding to 81-100 sequence of the encephalitogenic mouse MBP epitope)

The peptide compounds were synthesized by solid phase synthesis following the Fmoc/DCC/HOBt methodology (Bodanszky, "Principles of peptide synthesis", Springer-Verlag, New York, 1984) on a fully automated Model 431A Applied Biosystem peptide synthesizer, software version 1.2, following the manufacturer instruction. The synthesis was carried out on the acid labile resin (Wang resin) (NOVABIOCHEM, Laufelfinger, CH) for peptide synthesis pre-derivatized with Fmoc-Glycine (NOVABIOCHEM, Laufelfinger, CH No. Cat. 04-12-2010, 0.1 mmole) for the synthesis of the peptide compound of formula (I) (SEQ ID NO: 1) and pre-derivatized with Fmoc-glutamine (trytil) (NOVABIOCHEM, Laufelfingen, CH No. Cat. 04-12-2036, 0.1 mmol) for the synthesis of the peptide compound P81-100 (SEQ ID NO: 5). The N-terminal Fmoc protected amino group has been removed in the first synthesis cycle by treatment with 3 mL of 20 % pyridine in N-methyl-pyrrolidone (Merck, Darmstadt, Germany) for 14 minutes at room temperature under agitation. The deprotected resin has been then washed 5 times with N-methyl-pyrrolidone (2.5 mL) for 9 minutes at room temperature. In the mean time, the second amino acid to be added (1 mmole) has been activated at the carboxyl end by treatment with 1 mL of 1 M HOBT in N-methyl-pyrrolidone and 1 mL of 1 M dicyclohexylcarbodiimide (DCC) in N-methyl-pyrrolidone. The activated amino acid has been then incubated with the resin for 51 minutes under agitation. The resin has been then washed four times with 2 mL of N-methyl-pyrrolidone for 0.5 minutes. At this point the resin has been deprotected with piperidine and subjected to a subsequent coupling step with the next amino acid. This procedure has been repeated in a sequential manner for all the amino acids protected as indicated below:

Fmoc-Gly-OH, Fmoc-Ser(tBu)-OH, Fmoc-Gln(Trt)-OH, Fmoc-Thr(tBu)-OH, Fmoc-His(Trt)-OH, Fmoc-Val-OH, Fmoc-Lys(Boc)-OH, Fmoc-Pro-OH, Fmoc-Arg(Pbf)-OH, Fmoc-Asn(Trt)-OH, Fmoc-Phe-OH, Fmoc-Ile-OH. After completion of the synthesis, and after removal of the N-terminal Fmoc group with piperidine, the resin has been washed with methanol, dichloromethane, and again with methanol (MERCK, Darmstadt, Germany) and accurately dried under vacuum for 12 hours. Then the peptidic material has been detached from the resin by treatment of 200 mg of the resin with 5 mL of a mixture of trifluoroacetic acid (Pierce, Rockford, IL, USA), phenol (Sigma-Aldrich, Milan, Italy) thioanisol (Sigma-Aldrich, Milan, Italy) water (Sigma-Aldrich, Milan, Italy) and ethandithiol (Sigma-Aldrich, Milan, Italy) in the ratio 83:6:5:4:2 at room temperature for 3.5 hours. The mixture has been then filtered on a sintered glass funnel and the peptidic material precipitated by the addition of 10 mL of cold ethyl ether. The peptidic material has been obtained by centrifugation and the pellet has been dissolved in 25 mL of a mixture of water, acetonitrile (Merck, Darmstadt, Germany), and trifluoroacetic acid in the ratio 90:10:0.1, and purified by semipreparative RP-HPLC on a Aquapore column (Applied Biosystems, Foster City, CA, USA). Material corresponding to the main peak has been collected, frozen, and lyophilized. The peptide compounds were characterized by analytical RP-HPLC, amino acid analysis, and by determination of peptide resins were dried overnight under vacuum. Peptides compounds were cleaved from resin using TFA/phenol/thioanisole/H₂O/ethanedithiol 83:6:5:4:2 (5 mL/200 mg of resin), and incubating at room temperature for 3.5 h. The mixtures were then filtered and the peptidic material was precipitated by adding 10 mL of cold ethyl ether. After lyophilization the products were dissolved in H₂O/CH₃CN/TFA 90:10:0.1 and purified by semipreparative RP-HPLC. After purification the peptide were characterized by analytical HPLC, amino acid analysis and TOF-MALDI mass spectrometry on a KRATOS Kompact MALDI III (KRATOS Analyticals, Manchester, UK) using sinapinic acid as the matrix (Sigma-Aldrich, Milan, Italy).

The following peptide compounds were thus prepared:

### Assay 1

### In vivo activity of the peptide compound of formula (I) (SEQ ID NO: 1)

The *in vivo* activity of the peptide compounds of formula (I) (SEQ ID NO: 1), were evaluated on four groups of SJL female mice, used at the age of 6-15 weeks. This strain of mice has been genetically selected for its ability to develop experimental allergic encephalitis (EAE).

Two groups of mice have been immunized intraperitoneally with the peptide compound of formula (I) (SEQ ID NO: 1) (300 nmoles), dissolved in 0.05 ml of physiological solution and then emulsified with 0.05 ml of incomplete Freund's adjuvant (IFA) (Sigma, Italy).

As positive and negative control, P81-100 (SEQ ID NO: 5) and IFA alone were used respectively.

After two weeks EAE was induced in all groups by challenge with 300 nmoles of P81-100 (SEQ ID NO: 5) dissolved in 0.1 ml of physiological solution and then emulsified with 0.1 ml of complete Freund's adjuvant (CFA) (Sigma, Italy).

Pertussis toxin (200 ng) (Fluka Chemie AG, Switzerland) was injected intravenously at the time of immunization and 2 days later. The role of pertussis toxin is to enhance the migration of autoreactive lymphocytes through the encephalic barrier (Bernard, et al., "J. Immunol." 114, 1537-1540, 1975).

Mice were observed daily beginning at day 9 up to 60 for clinical signs of EAE and were graded according to the following clinical scale: 1, limp tail; 2, partial hind limp paralysis; 3, complete hind limp paralysis; 4, hind and front limp paralysis; 5, moribund (Broke S. et al., "Nature", 379, 343-346, 1996). Grade 5 mice were euthanized.

The results are shown in the Table 1.

| Peptide (nmol) | Incidence of EAE * | Day of on-set** | Severity*** |
|---|---|---|---|
| P81-100 (300) | 2/17 | 33±4.24 | 1.0±0 |
| IFA | 4/11 | 18.0±2.0 | 3.2±1.3 |
| Peptide I | 0/17 | - | - |

| | | | |
|---|---|---|---|
| * Incidence of EAE is expressed as number of mice with clinical EAE/number of mice immunized. | | | |
| ** The day of onset is expressed as mean day of onset ± SD (Standard Deviation). | | | |
| *** The severity is expressed as mean severity of sick mice ± SD. | | | |

Data from Table I shows that mice treated with the peptide compound of formula (I) (SEQ ID NO: 1) didn't develop EAE.

## Claims

1. A peptide compound of formula (I) :
where
R is H- or COCH₃, R' is COOH or CONH₂ and
each amino acid has L or D configuration.

2. A pharmaceutical composition comprising an effective dose of a peptide compound according to claim 1, and
at least a pharmaceutical acceptable inert ingredient.

## Patentansprüche

1. Peptidverbindung der Formel (I):
worin
R für H- oder COCH₃ steht, R' für COOH oder CONH₂ steht und jede Aminosäure L- oder D-Konfiguration besitzt.

2. Pharmazeutische Zusammensetzung, umfassend eine wirksame Dosis einer Peptidverbindung nach Anspruch 1 und wenigstens einen pharmazeutisch akzeptablen inerten Inhaltsstoff.

## Revendications

1. Composé peptidique de formule (I) :
où
R est H-ou COCH₃, R' est COOH ou CONH₂ et
chaque aminoacide a la configuration L ou D.

2. Composition pharmaceutique comprenant une dose efficace d'un composé peptidique selon la revendication 1 et au moins un ingrédient inerte pharmaceutiquement acceptable.
